# EUROPEAN PATENT SPECIFICATION

(11) **EP 1 641 528 B1**
(45) Date of publication and mention of the grant of the patent: **31.03.2010**
(21) Application number: 04755266.6
(22) Date of filing: 15.06.2004
(51) Int. Cl.: A61N 1/362, A61N 7/00, A61N 1/39

(54) **VIBRATIONAL PACING THERAPY DEVICE**
VIBRATIONS-SCHRITTMACHER-THERAPIE-VORRICHTUNG
DISPOSITIF DE THERAPIE VIBRATIONNEL D'ENTRAINEMENT

(30) Priority: 17.06.2003 US 479347 P; 18.08.2003 US 496184 P; 18.08.2003 US 496179 P; 30.09.2003 US 507719 P; 06.11.2003 US 518138 P; 10.12.2003 US 528940 P; 10.12.2003 US 528939 P
(43) Date of publication of application: 05.04.2006
(73) Proprietor: EBR Systems, Inc., Sunnyvale, California 94085 (US)
(72) Inventor: ECHT, Debra, S., Woodside, CA 94062 (US); BRISKEN, Axel, F., Fremont, CA 94539 (US); RILEY, Richard, E., Palo Alto, CA 94303 (US); COWAN, Mark, W., Fremont, CA 94539 (US)
(74) Representative: Kazi, Ilya
(86) International application number: PCT/US2004/018987
(87) International publication number: WO 2004/112895

(56) References cited:
- EP-A- 0 617 981
- WO-A-00/42914
- WO-A-00/62858
- US-A- 3 857 382
- US-A- 4 265 228
- US-A- 5 935 158
- US-A- 5 935 158

## Description

### BACKGROUND OF THE INVENTION

### 1. Field of the Invention.

The systems and methods of this invention relate to pacing treatment of the heart comprising applying vibrational energy.

Heart Failure (HF) currently affects over 5 million patients in the United States alone. This population has been steadily increasing due to overall demographic aging and, in particular, the effects of new life-prolonging treatments to patients with chronic cardiac conditions. HF is defined by ACC/AHA Task force as a complex clinical syndrome that impairs the ability of the ventricle to fill with or eject blood. New medications developed to treat HF have been generally ineffective, and device-based solutions appear to present a significant opportunity for afflicted patients.

HF generally results from one or more underlying factors including hypertension, diabetes, valvular disease, cardiomyopathy, coronary artery disease, or structural changes to the heart muscle. HF is characterized by reduced ventricular wall motion in systole and/or diastole, and low ejection fraction. As the heart becomes less able to pump sufficient volume to the system, patients develop symptoms of fluid retention, shortness of breath, and fatigue.

Approximately one third of patients with HF have poor timing of contraction between the right and the left ventricle and within the left ventricle, called interventricular and intraventricular dyssynchrony, respectively. This is sometimes also manifest by a wider than normal QRS interval on a surface electrocardiogram (ECG) taken of a HF patient. The wider than normal QRS interval is called conduction delay because there is a prolonged time interval for the normal electrical impulse to travel ("conduct") to all parts of both ventricles. This is also sometimes manifest by conduction delay between the atria and ventricles (A-V delay). Ventricular dyssynchrony and conduction delays can contribute to weak left ventricular function by causing delayed and/or abnormal left ventricular contraction. There may be inadequate filling and emptying of the left ventricle, as well as backflow of blood into the left atrium, resulting in decreased cardiac output and increased symptoms for the patient. This dysfunction causes increased mortality and morbidity among patients with HF.

Cardiac resynchronization therapy is the use of pacing to coordinate the contraction of the ventricles in order to reduce heart failure and improve prognosis in HF patients. Recently, devices that pace both ventricles, referred to as bi-ventricular pacing, have been adopted to provide cardiac resynchronization therapy. A bi-ventricular pacing system utilizes conventional dual chamber, right atrium and right ventricle, pacing technology but adds a third lead, usually in a coronary vein, to sense and pace the epicardial surface of the left ventricle. The pacing device can then, at an appropriate time interval after right atrial activity, synchronize contraction of both right and left ventricles either simultaneously or at coordinated time intervals. The synchronous contraction of the ventricles facilitates more adequate filling of the left ventricle and less backflow (mitral valve regurgitation to the left atrium), resulting in more oxygenated blood being pumped to the body. Alternatively, it has been shown that pacing only the left ventricle at a location near the apex is associated with improvement in left ventricular function. However, this location is not accessible from the coronary veins in current pacing systems.

Clinical studies have shown a sustained improvement of symptoms and exercise tolerance in patients using bi-ventricular pacing devices to improve left ventricular function. Cardiac resynchronization therapy has also been incorporated into implantable cardioverter defibrillator (ICD) devices, allowing for the simultaneous treatment of heart failure and the prevention of sudden cardiac death caused by life-threatening ventricular arrhythmias in HF patients.

Pacemaker leads are typically placed through the skin into a subclavian vein to access the venous side of the cardiovascular system. In bi-ventricular pacing systems, one lead is placed in contact with the right ventricular wall and one lead is placed in contact with the right atrial wall. To access the left ventricle, the third lead is passed into the right atrium, into the orifice of the coronary sinus, and then maneuvered through the coronary veins to a position on the epicardial aspect of the lateral wall of the left ventricle. Some work has been done exploring minimally invasive methods of alternatively placing the lead/electrode directly on the epicardium of the left ventricle.

Placement of the third lead to contact the left ventricle has been a significant problem for application of this therapy. The coronary sinus is a complicated venous pathway with multiple branches which bend and narrow with considerable variation as they extend distally onto the epicardium of the left ventricle. Placement of this lead requires significant skill an the part of the physician. In order to provide adequate steerability and pushability, the design of the left ventricular lead or a lead introduction system/device is much more complicated than for regular pacing leads. Often the left ventricular lead positioning/placement can take over an hour to perform exposing the patient to increased fluoroscopy radiation and increased procedure risks. Furthermore, in some patients (7.5% in the MIRACLE study), an acceptable lead placement is not possible due to anatomic constraints or undesirable phrenic nerve pacing. Additionally, lead dislodgement and loss of pacing capture have been a common complication in the use of these coronary sinus leads (e.g., 10-20% complication rates have been reported within the first 6 months of device placement).

It would be beneficial to eliminate the third pacing lead and yet provide resynchronization within the left ventricle and/or between the left and right ventricles.

Moreover, it would be beneficial to provide more physiological pacing of the right ventricle.

In normal physiology, the right ventricle is first stimulated in the upper septal area, and then the impulse travels down specially conducting pathways to the right ventricular apex.

However, pacing from the right ventricle is virtually always accomplished from a lead tip located in the right ventricular apex, such that the conduction pathway is abnormal and slow.

Clinical trials have recently shown that in patients with and without A-V block, pacing from the right ventricular apex can result in increased total mortality and re-hospitalization for heart failure compared to non-paced patients. The possible adverse effects of pacing the right ventricular apex in patients without bi-ventricular pacemakers is unknown, but a source of growing concern.

### 2. Description of the Background Art.

This application has disclosure related to prior commonly assigned provisional applications 60/479,347 (Attorney Docket No. 21834-000100US), filed on June 17, 2003; 60/496,184 (Attorney Docket No. 21834-000110US), filed on August 18, 2003; 60/496,179 (Attorney Docket No. 21834-000200US), fled on August 18, 2003; and 60/507,719 (Attorney Docket No. 21834-000300US), filed on September 30, 2003.
U.S. Patent: 4,928, 688/RE38,119 Mower, Method and apparatus for treating hemodynamic dysfunction.
U.S. Patent 5,174, 289 Cohen: Pacing systems and methods for control of the ventricular activation sequence.
U.S. Patent: 5,018,523 Bach et al.; Apparatus for common mode stimulation with bipolar sensing.
U.S. Patent: 6.070,101 Struble et al.; Multiple channel, sequential, cardiac pacing systems.
U.S. Patent 6,439,236 Porter and Xie; Methods of inducing atrial and ventricular rhythms using ultrasound and microbubbles.
PCT WO 03/070323 Adam et al; Ultrasound Cardiac Stimulator.
U.S. Patent: 6,223,079 Bakels et al.; Bi-ventricular pacing method.
U.S. Patent 4,651,716 Forester et al.; Method and device for enhancement of cardiac contractility.
PCT WO 9961058 Van der Wouw; Method of altering heart beat.
ACC/AHA Task Force on Practice Guidelines. Evaluation and Management of Chronic Heart Failure in the Adult. JACC 2002;38:2101-13.
Daubert et al., "Use of Specifically Designed Coronary Sinus Leads for Permanent Left Ventricular Pacing: Preliminary Experience", PACE, 1997; 20: II- NASPE Abstract 17, Apr., 1997.
Leclerq C et. al., "Acute Hemodynamic Effects ofBiventricular DDD Pacing in Patients with End-Stage Heart Failure", JACC 1998;32:1825-1831.
Daubert et al., "Permanent Left Ventricular Pacing With Transvenous Leads Inserted Into The Coronary Veins", PACE 1998;21;239-245.
Daoud et al., "Implantation Techniques and Chronic Lead Parameters of Biventricular Pacing Dual-chamber Defibrillators", J Cardiovasc Electrophysiology 2002; 13:964-970.
Valls-Bertault et al., "Adverse Events with Transvenous Left Ventricular Pacing in Patients with Severe Heart Failure: Early Experience from a Single Centre", Europace 2001;3:60-63.
Leclercq C et al., "Systolic Improvement and Mechanical Resynchronization does not Require Electrical Synchrony in the Dilated Failing Heart with Left Bundle-Branch Block", Circulation 2002;106:1760-1763.
Linde C et al., "Long-Term Benefits of Biventricular Pacing in Congestive Heart Failure: From the Multisite Stimulation In Cardiomyopathy (MUSTIC) Study", J Am Coll Cardiol 2002;40:111-118.
Abraham WT et al., "Cardiac Resynchronization in Chronic Heart Failure", N Engl J Med 2002;346:1845-1853.
Bradley DJ et al., "Cardiac Resynchronization and Death from Progressive Heart Failure: A Meta-Analysis of Randomized Controlled Trials," JAMA 2003;289:730-740.
Nielsen JC et al., "A Randomized Comparison of Atria and Dual-Chambered Pacing in 177 Consecutive Patients with Sick Sinus Syndrome," J Am Coll Cardiol 2003;42:614-623.
DAVID Trial Investigators, "The Dual Chamber and VVI Implantable Defibrillator (DAVID) Trial," JAMA 2002;288:3115-3123.
MIRACLE Trial Investigators, "Combined Cardiac Resynchronization and Implantable Cardioversion Defibrillation in Advanced Heart Failure: the MIRACLE ICD Trial," JAMA 2003;289:2685-2694.

EP-A-0617981 describes an implantable heart defibrillator with a shock pulse generator for delivering a defibrillation shock to a heart where the delivered energy is mechanical. The shock pulse generator consists of an electro-mechanical energy convertor which generates, for example by means of a piezo-element, pressure in a pressure chamber. The pressure is transmitted in a fluid to a balloon-like head in contact with heart tissue. The generated pressure causes the head to expand and deliver a mechanical shock to the heart corresponding to the transmitted pressure. US-A-5935158 describes an implantable piezo-electric electrode for electrically and mechanically stimulating tissue and for detecting electrical or mechanical evoked response of the simulated tissue. WO 00/42914 describes apparatus and methods for in vivo chronic measurement of cardiac monophasic action potentials.

### BRIEF SUMMARY OF THE INVENTION

According to the present invention, there is provided an implantable pacing system as set out in Claim 1. For this invention, the use of a device to effect left ventricular pacing and/or to synchronize left ventricular pacing with right ventricular activation provides an improved method of treating patients with heart failure or possibly of preventing heart failure. The improvement uses vibrational energy to effect left ventricular pacing to the left ventricle without the use of an implanted intracardiac or epicardial lead in contact with the left ventricle. Optionally, a system of the present invention may rely on delivery of the vibrational energy from an external source to provide temporary left ventricular pacing treatment for heart failure. The system described is a fully implanted subcutaneous device that provides ultrasound energy at frequencies, amplitudes, and treatment durations that stimulate cardiac tissue without the use of leads contacting left ventricular tissue.

A treatment regime for providing synchronized beating of the left and right ventricle is accomplished in part by applying a vibrational energy wave. The vibrational wave stimulates the heart. Once stimulated, a QRS complex can be seen on an electrocardiogram and contraction of the heart chamber (s) is initiated. In this invention, the wave will either a) simultaneously stimulate both ventricles to contract, b) stimulate the ventricles in a preferred, more physiologic, conduction pattern, or c) be delivered in coordination with an electrical pacing and sensing lead, such that one ventricle is electrically paced and the vibrational wave stimulates the other ventricle. Vibrational energy offers the potential benefit of being able to stimulate without tissue contact, and, therefore, is not limited to the right ventricular apex as a pacing site nor does it require direct placement and contact of a lead in or on the left ventricle.

The vibrational wave can be applied to stimulate each heart beat with ultrasound as a single burst or as multiple bursts with appropriate selection of the following parameters

| Parameter | Value Range |
|---|---|
| Ultrasound Frequency | 20KHz - 5MHz |
| Burst Length (#cycles) | 3-250 |
| Pace Pulse Duration | 0.12µS - 13mS |
| Duty Cycle | 0.1-100% |
| Intensity | >0.2W/cm² |

The device would contain one or more ultrasound transducers of appropriate size and aperture to stimulate heart tissue within the ultrasound beam. The transducer portion of the device would be implanted subcutaneously in the anterior chest surface of the body in such fashion as to target the desired heart tissue within the beam profile of the transducer. The beam profile would need only target a sufficient volume of tissue to generate a vibrationally-induced paced beat. If the tissue volume required for stimulation is small, a narrow beam could used. However, a wide beam could also be used and could successfully stimulate multiple chambers simultaneously. Furthermore, multiple beams could be utilized to stimulate multiple sites within the heart, either simultaneously or sequentially per a programmable delay function.

In a combined electrical pacing and vibrational pacing device, the synchronization of the delivery could be accomplished either within a single enclosure or in two separate enclosures. Separate enclosures would require communication between the devices to synchronize the beats or detection of an electrically-paced beat by one device and an immediate vibrationally-paced beat response by the other device, or vice-versa.

As in all pacemaker devices, which include a variety of pacing modalities, the delivery of the vibrational pacing energy could be triggered based on sensed or programmed heart rates or inhibited by sensed cardiac events in the atrium or ventricle. When used for biventricular pacing, with vibrational pacing of the left ventricle in combination with electrical pacing of the right ventricle, the vibrational energy would be triggered to be synchronous, i.e. simultaneous or at a programmable delay, from the right ventricle electrically-paced beat. Fundamentally, if a right ventricular beat is triggered or sensed by the vibrational device, then vibrational energy is delivered to the left ventricle to synchronize the chambers.

In the simplest form, the device would contain a vibrational energy delivery mechanism to stimulate the left ventricle. It would provide a fixed programmable heart rate that stimulates a paced beat of the left ventricle via vibrational energy. The paced beat would then normally conduct to the right ventricle. This would be analogous to a ventricular pacing and sensing (with inhibition) referred to as a VVI pacing modality. An enhanced pacing modality referred to as VVI/T also includes programmability to trigger a paced beat in response to a sensed beat.

In a more complex form, the device would contain multiple vibrational energy mechanisms to stimulate the heart tissue at multiple points, e. g. , at multiple locations within the left ventricle, and/or within both the left and right ventricles. Stimulation could occur either simultaneously or sequentially, per a programmable function.

In the most complex form, the device would contain a vibrational energy delivery mechanism for left Ventricular stimulation and also contain electrical capabilities for pacing and sensing of both right atrial and right ventricular chambers with programmable capabilities for all combinations of pacing modalities (e. g. DDDR+, Dual chamber pacing, Dual chamber sensing, Dual chamber triggered and inhibited modes with Rate responsive sensors and mode adaptation). Optionally, the device would contain the capability for high energy delivery used for cardioversion and defibrillation, using either electrical energy or vibrational energy.

A method is described for pacing the heart, the method comprising directing vibrational pacing energy to at least a portion of ventricular tissue of the heart, wherein the vibrational energy stimulates contraction of at least one ventricle. In one example, the vibrational energy is selectively directed at left ventricular tissue. In one example, the vibrational energy is selectively directed at right ventricular tissue. In one example, the vibrational energy is directed at both left and right ventricular tissues. The vibrational pacing energy may be directed as one or more narrow beams or as one or more wide beams or as any combination of one or more narrow or wide beams. The vibrational pacing energy may be delivered from a single implanted enclosure, from two or more implanted enclosures or from an external vibrational transducer. Delivery of the vibrational energy may be programmed to promote synchronized contraction of the left and right ventricles, In an example, a method further comprises electrically stimulating one or more regions of the heart in a coordinated pattern with the delivery of the vibrational pacing energy. In an example, a method further comprises detecting the presence or absence of cardiac signals originating in the ventricles or atria of the heart and triggering or inhibiting the delivery of electrical or vibrational energy based on programmed parameters. In an example, a method further comprises directing vibrational pacing energy at one or more regions of the left ventricle, wherein the vibrational energy selectively stimulates the left ventricle. In an example, a method further comprises stimulating at least the right ventricle. In an example, a method further comprises stipulating the right atrium. In an example the right ventricle and/or the right atrium is (are) stimulated with vibrational energy. In an example, the right ventricle and/or the right atrium is (are) stimulated with electrical energy. In an example a method further comprises the vibrational pacing energy and/or the electrical pacing energy are delivered in a pattern selected to promote synchronized contraction of the left and right ventricles. In an example a method further comprises detecting the presence or absence of cardiac signals originating in the ventricles or atria of the heart and triggering or inhibiting electrical or vibrational energy based on programmed parameters. In an example, the vibrational pacing energy is delivered as single or multiple bursts having a frequency from 20 kHz to 5 MHz, a pulse duration from 0.12 ysec to 13 msec, a duty cycle from 0.1% to 100%, and an intensity greater than 0.2 W/cm2.

A pacing system is described comprising: an implantable enclosure having circuitry for controlling a vibrational transducer under conditions selected to stimulate heart contraction when directed at cardiac tissue. In an example, the circuitry comprises a power amplifier, an impedance matching circuit, and a signal generator for controlling the vibrational transducer. In an example, the system further comprises circuitry for generating electrical pulse (s) for stimulating heart contraction and circuitry for detection of intrinsic cardiac signals. In an example, the system further comprises circuitry which analyzes detected intrinsic heart signals and which controls the electrical pulses and vibrational pacing to synchronize contraction of the left and right ventricles. In an example. the system further comprises the electrical pulse circuitry and detection circuitry is located in the same enclosure as the vibrational transducer circuitry. In an example, the vibrational transducer is located within the enclosure. In an example, the vibrational transducer is connected to the enclosure by a cable. In an example, one or more vibrational transducers are located on a lead and positioned In the right ventricle. In an example, one or more transducers are located on a lead and positioned in a subcutaneous location. In an example, one or more transducers are located on a lead and positioned in the right atrium. In an example, a vibrational transducer is directed to ventricular tissue. In an example, a vibrational transducer is directed to atrial tissue. In an example, a vibrational transducer is directed to both atrial and ventricular tissue. In an example, the enclosure is adapted to include a cardioverter defibrillator. In an example, the cardioverter defibrillator uses electrical energy. In an example, the cardioverter defibrillator uses vibrational energy.

A pacing system is described comprising: a vibrational transducer; and control circuitry for activating the vibrational transducer in order to deliver controlled vibrational energy to a target region of the heart under conditions selected to promote synchronized contraction of the ventricles. In an example, the vibrational transducer is adapted to contact an exterior surface of the patient's skin and deliver the vibrational energy through the tissue overlying the target region of the heart. In an example, the control circuitry comprises a power amplifier, an impedance matching circuit, and a signal generator for activating the vibrational transducer. In an example, the system further comprises control circuitry which analyzes detected intrinsic heart signals and which controls the vibrational energy to promote synchronized contraction of the ventricles.

### BRIEF DESCRIPTION OF THE DRAWINGS

Figures 1A and 1B illustrate two embodiments with one or more right-sided transvenous leads; a left-sided transducer over or between the ribs with a left-sided canister implant (Fig. 1A), and a medial transducer placement over the sternum with a right-sided canister implant (Fig. 1 B).
Figure 2A and 2B illustrate an alternative embodiment without leads and a canister housing the ultrasound transducer implanted in the left precordial subcutaneous space.
Figures 3A and 3B illustrate an alternative embodiment with one or more subcutaneous leads attached to a canister implanted in the left precordial subcutaneous space.
Figure 4 illustrates an alternative embodiment with two right-sided transvenous leads, a ventricular lead incorporating a transducer within the lead body and an atrial lead, and a canister in the right subcutaneous space.
Figure 5 illustrates a lead design incorporating an electrode pair at the distal end for pacing and sensing, and one or more transducers within the body of the lead.
Figure 6 is a block diagram showing an embodiment of the control circuitry implementation of the present invention.

### DETAILED DESCRIPTION OF THE INVENTION

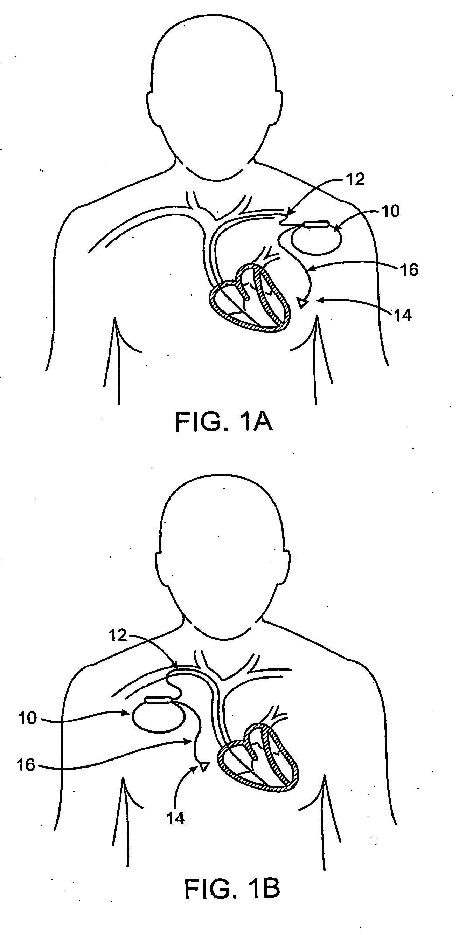

In one exemplary embodiment, all the device sensing, logic and energy source components are housed within a single canister 10 implanted beneath skin and adipose tissue in the left (Figure 1A) or right (Figure 1B) subclavian region of the chest wall. In this embodiment, electrical leads 12 containing electrodes are passed transvenously through the superior vena cava into the right atrium and/or right ventricle. An ultrasound transducer 14 is separately connected to the canister by a cable 16. The transducer is encased in an appropriate housing and is subcutaneously implanted over the ribs, over the sternum, or between the ribs in order to target the preferred region of the left or right ventricle within the ultrasound beam profile. The connecting cable 16 is tunneled subcutaneously to the canister and connected.

Alternatively, two canisters may be implanted subcutaneously beneath skin and adipose tissue (not shown). The first canister houses the device sensing, logic and energy source components required for the electrical pacemaker/cardioverter/defibrillator and may be implanted on the left or right subclavian regions. The second canister is located in the left anterior chest region over the ribs or between the ribs or it is located over the sternum. The second canister houses the transducer, device sensing, logic, and energy source for pacing using vibrational energy. A connecting cable is tunneled subcutaneously between the two canisters.

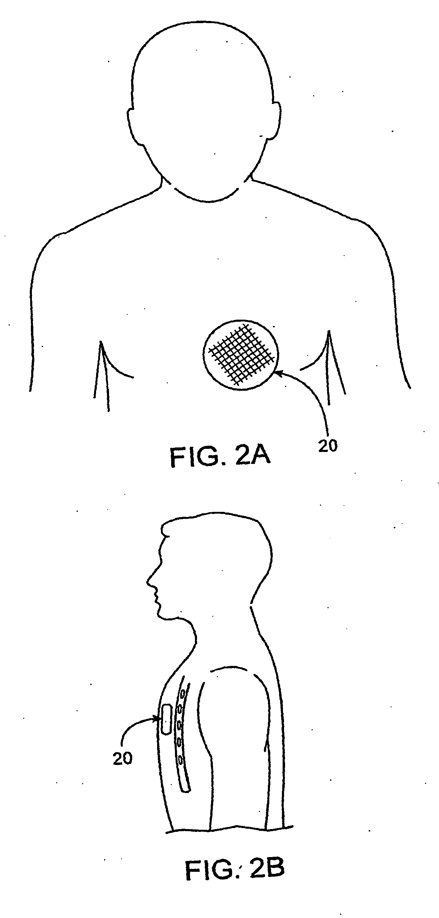

In another embodiment, (Figures 2A and 2B), the device 20 is a single canister with no transvenous leads. As previously disclosed the device can be subcutaneously implanted in the left anterior chest region or over the sternum with the ultrasound beam(s) directed to the ventricle(s) from a transducer within the canister. This represents a programmable rate VVI/T device that paces only the left ventricle or simultaneously or sequentially paces the left and right ventricles using vibrational energy. An electrocardiogram sensing circuit would preferably be provided in this embodiment with electrodes on the surface of the canister, and would provide either an inhibited pacing operation with a detected ventricular beat or a synchronized pacing operation with a detected ventricular beat.

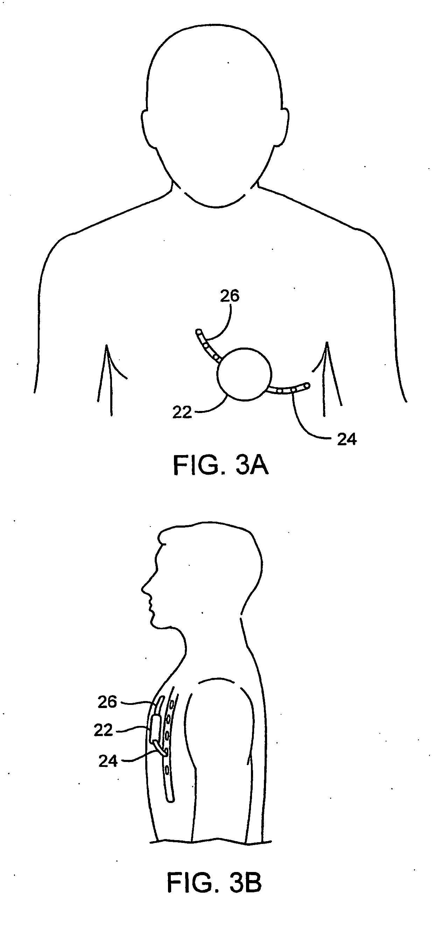

In another embodiment, (Figures 3A and 3B), the device 22 is a single canister housing the sensing, logic, and energy source components for pacing using vibrational energy. One or more subcutaneous leads 24 and 26 containing one or more vibrational energy elements arranged linearly or in another pattern are connected to device 22. Electrodes for sensing of the electrocardiogram (sensors) are provided on either or both leads 24 and 26 or the surface of the canister. This represents an alternative programmable rate VVI/T device that paces either the left ventricle or simultaneously or sequentially paces the left and right ventricles using vibrational energy.

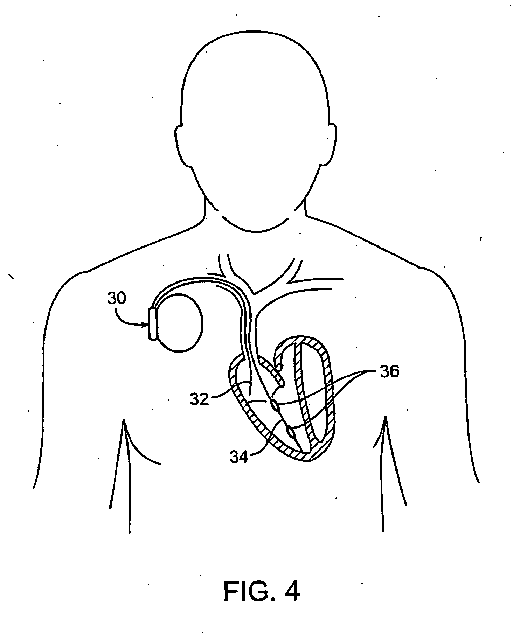

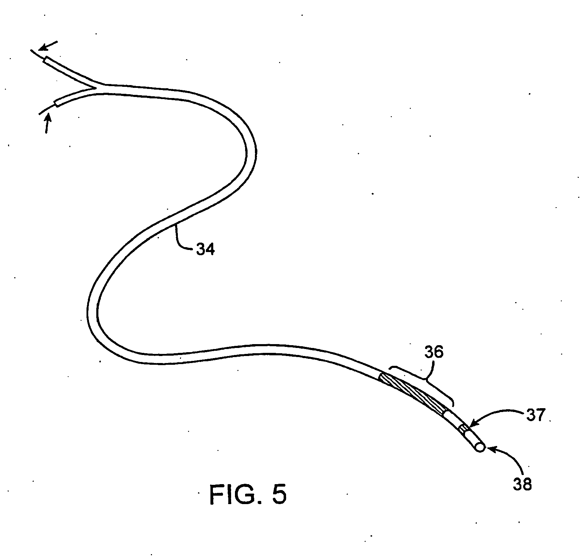

In another embodiment (Figure 4), the device 30 is a single canister with transvenous lead 32 containing capability for electrical sensing and pacing and transvenous lead 34 containing capability for electrical sensing and pacing and vibrational pacing. The single canister 30 would be implanted beneath skin and adipose tissue in the left or right subclavian region. In this embodiment, the right ventricular lead 34 (Figure 5) containing both electrical and vibrational energy components, and a right atrial lead 32 containing electrical energy components are utilized. The leads are passed transvenously through the superior vena cava into the right ventricle and the right atrium. In this embodiment transducer(s) 36 would be contained within the body of the right ventricular lead. The transducer(s) 36 would deliver vibrational energy to pace one or both ventricles. The electrical component of the right ventricular lead would primarily be used for sensing, but could optionally be used for electrical pacing. The right atrial lead would be used for both sensing and pacing.

Another embodiment would be similar to Figure 4, except that both the right atrial lead 32 and right ventricular lead 34 would contain both electrical 37 and 38 and vibrational 36 energy components as shown in Figure 5. In this embodiment, the right atrial lead would function in a manner similar to the right ventricular lead, to accomplish pacing and sensing of the right and left atria.

Alternatively, the right atrial lead 32 would not be present, and the right ventricular lead would be as shown in Figure 5 with an added electrical sensing electrode (not shown) located on a proximal portion of the lead such that the electrode would be positioned within the right atrium.

Alternatively, the right atrial lead would not be present, and the right ventricular lead would be as shown in Figure 5 with an added electrical sensing electrode (not shown) and with an added vibrational energy transducer (not shown) located on a proximal portion of the lead such that the components would be positioned within the right atrium. In this embodiment a single lead could provide pacing and sensing of the right and left atria and separately pacing and sensing of the right and left ventricles.

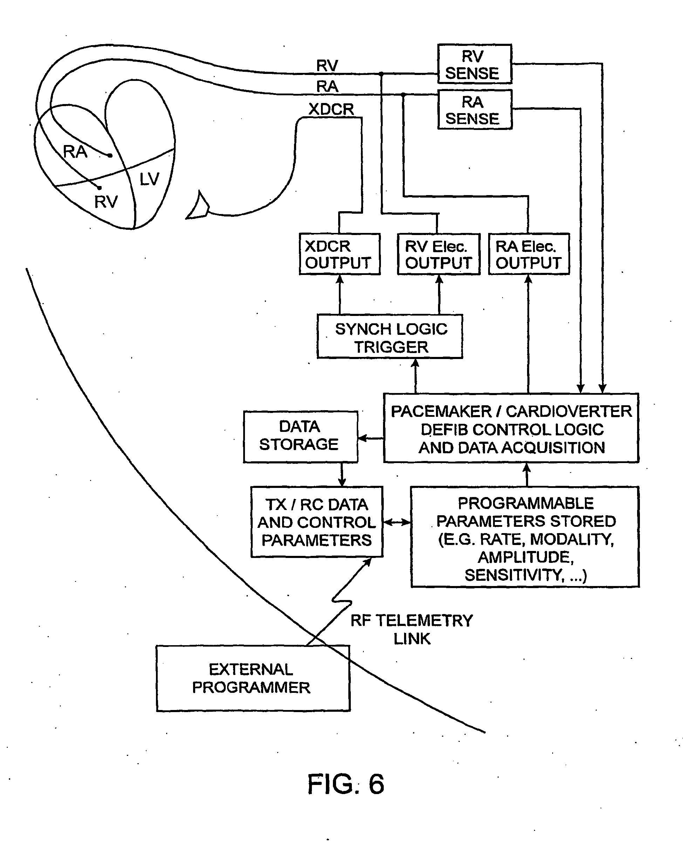

Figure 6 provides a block diagram of circuitry for implementing the most complex version of the device including dual chamber sensing, dual chamber electrical pacing, electrical cardioversion and defibrillation, and vibrational energy pacing. Alternatively, the cardioversion and defibrillation may be provided by vibrational energy.

The device designs and implementations referred to thus far are generally useful for the treatment of patients with heart failure. The treatment of heart failure, however, may be accomplished with systems which may be somewhat simpler that those described above to promote temporary synchronized contraction of the ventricles. In particular, the vibrational transducers may be adapted for manual control by either the patient or by a doctor or other medial personnel. Most simply, the vibrational transducer may be incorporated into external units capable of being applied to the anterior chest (not shown). Usually, the patient will be reclining on the table or bed, the vibrational transducer, attached by a cable to an external generator, is applied over the patient's chest, preferably using a gel layer to enhance contact. Usually, the transducer will be placed generally over the ventricular region of the heart and the transducer may be configured to direct energy over specific ventricular regions.

In embodiments, the circuitry comprises a power amplifier, an impedance matching circuit, and a signal generator for controlling the vibrational transducer.

Systems embodied for external use have sensor circuitry, control circuitry, power supply, and burst generation incorporated into the generator (not shown). The ECG sensors may be incorporated into the transducer housing or optionally standard transcutaneous electrodes may be connected to the body and to the generator via cables. Alternatively, the generator may accept ECG signals directly from an external electrocardiogram system. Intrinsic heart signals detected from ECG sensors are analyzed by control circuitry and are used to control pacing using the vibrational energy as discussed above for implantable systems.

## Claims

1. An implantable pacing system for the treatment of heart failure, comprising:
at least one implantable enclosure (10; 20; 22; 30) housing circuitry configured to (a) control electrical stimulation pulses for initiating heart contraction, wherein the pulses are delivered using one or more pacing leads, (b) analyze detected cardiac signals, wherein the cardiac signals are detected using one or more sensing leads, and (c) activate transmission of vibrational energy that is generated by one or more implantable transducers (14; 36) to pace the heart, wherein the circuitry is adapted to control the electrical stimulation pulses and the transmission of vibrational energy under conditions selected to synchronize contraction of the heart's chambers.

2. The pacing system as in claim 1, wherein the circuitry comprises a first circuit configured to control the one or more electrical stimulation pulses, a second circuit configured to analyze detected cardiac signals, and a third circuit configured to activate transmission of vibrational energy.

3. The pacing system as in claim 1, wherein the at least one implantable enclosure comprises (a) a first enclosure (10) comprising the first and second circuits, and (b) a second enclosure comprising the third circuit and one or more vibrational transducers, wherein the first and second enclosures are connected by a cable.

4. The pacing system as in claim 3, wherein the third circuit comprises a power amplifier, an impedance matching circuit, and a signal generator configured to activate the one or more vibrational transducers.

5. The pacing system as in claim 3, wherein the second circuit further comprises circuitry configured to generate the electrical stimulation pulses and circuitry configured to detect cardiac signals.

6. The pacing system as in claim 5, wherein the electrical stimulation pulse circuitry and the detection circuitry are located in the first enclosure.

7. The pacing system as in claim 3, wherein at least one of the vibrational transducers is adapted to be directed to ventricular tissue.

8. The pacing system as in claim 3, wherein at least one of the vibrational transducers is adapted to be directed to atrial tissue.

9. The pacing system as in claim 3, wherein at least one of the vibrational transducers is adapted to be directed to atrial or ventricular tissue.

10. The pacing system as in claim 3, wherein at least one of the implantable enclosures is adapted to include a cardioverter defibrillator.

11. The pacing system as in claim 10, wherein the cardioverter defibrillator uses electrical energy.

12. The pacing system as in claim 11, wherein the cardioverter defibrillator uses vibrational energy.

13. The pacing system as in claim 9, further comprising one or more pacing and sensing leads implantable in the heart and connected to the first enclosure and connected to the first and second circuits.

14. The pacing system as in claim 13, wherein contraction is synchronized between the left and right ventricle.

15. The pacing system as in claim 13, wherein the contraction is synchronized between the right atrium and the left ventricle.

16. The pacing system as in claim 3, wherein the second enclosure is implantable in a subcutaneous location and the cable is locatable in a subcutaneous tunnel between the second enclosure and the first enclosure.

## Patentansprüche

1. Implantierbares Schrittmachersystem für die Behandlung eines Herzfehlers, das umfasst:
wenigstens ein implantierbares Gehäuse (10; 20; 22; 30), das eine Schaltungsanordnung aufnimmt, die konfiguriert ist, um (a) elektrische Stimulationsimpulse zum Einleiten einer Herzkontraktion zu steuern, wobei die Impulse unter Verwendung einer oder mehrerer Schrittmacherleitungen geliefert werden, (b) detektierte Herzsignale zu analysieren, wobei die Herzsignale unter Verwendung einer oder mehrerer Abfühlleitungen detektiert werden, und (c) die Übertragung von Schwingungsenergie zu aktivieren, die durch einen oder mehrere implantierbare Wandler (14; 36) erzeugt wird, um das Herz zum Schlagen anzuregen, wobei die Schaltungsanordnung dazu ausgelegt ist, die elektrischen Stimulationsimpulse und die Übertragung von Schwingungsenergie unter Bedingungen zu steuern, die gewählt sind, um die Kontraktion der Herzkammern zu synchronisieren.

2. Schrittmachersystem nach Anspruch 1, wobei die Schaltungsanordnung eine erste Schaltung, die konfiguriert ist, um den einen oder die mehreren elektrischen Stimulationsimpulse zu steuern, eine zweite Schaltung, die konfiguriert ist, um detektierte Herzsignale zu analysieren, und eine dritte Schaltung, die konfiguriert ist, um die Übertragung von Schwingungsenergie zu aktivieren, umfasst.

3. Schrittmachersystem nach Anspruch 1, wobei das wenigstens eine implantierbare Gehäuse umfasst: (a) ein erstes Gehäuse (10), das die erste und die zweite Schaltung enthält, und (b) ein zweites Gehäuse, das die dritte Schaltung und einen oder mehrere Schwingungswandler enthält, wobei das erste und das zweite Gehäuse durch ein Kabel verbunden sind.

4. Schrittmachersystem nach Anspruch 3, wobei die dritte Schaltung einen Leistungsverstärker, eine Impedanzanpassungsschaltung und einen Signalgenerator, der konfiguriert ist, um den einen oder die mehreren Schwingungswandler zu aktivieren, umfasst.

5. Schrittmachersystem nach Anspruch 3, wobei die zweite Schaltung ferner eine Schaltungsanordnung, die konfiguriert ist, um die elektrischen Stimulationsimpulse zu erzeugen, und eine Schaltungsanordnung, die konfiguriert ist, um Herzsignale zu detektieren, umfasst.

6. Schrittmachersystem nach Anspruch 5, wobei die Schaltungsanordnung für elektrische Stimulationsimpulse und die Detektionsschaltungsanordnung sich im ersten Gehäuse befinden.

7. Schrittmachersystem nach Anspruch 3, wobei wenigstens einer der Schwingungswandler dazu ausgelegt ist, auf ventrikuläres Gewebe gerichtet zu werden.

8. Schrittmachersystem nach Anspruch 3, wobei wenigstens einer der Schwingungswandler dazu ausgelegt ist, auf atriales Gewebe gerichtet zu werden.

9. Schrittmachersystem nach Anspruch 3, wobei wenigstens einer der Schwingungswandler dazu ausgelegt ist, auf atriales oder ventrikuläres Gewebe gerichtet zu werden.

10. Schrittmachersystem nach Anspruch 3, wobei wenigstens eines der implantierbaren Gehäuse dazu ausgelegt ist, einen Cardioverter-Defibrillator zu enthalten.

11. Schrittmachersystem nach Anspruch 10, wobei der Cardioverter-Defibrillator elektrische Energie verwendet.

12. Schrittmachersystem nach Anspruch 11, wobei der Cardioverter-Defibrillator Schwingungsenergie verwendet.

13. Schrittmachersystem nach Anspruch 9, das ferner eine oder mehrere Schrittmacher- und Abfühlleitungen umfasst, die im Herz implantierbar sind und mit dem ersten Gehäuse sowie mit der ersten und der zweiten Schaltung verbunden sind.

14. Schrittmachersystem nach Anspruch 13, wobei die Kontraktion zwischen dem linken und dem rechten Ventrikel synchronisiert ist.

15. Schrittmachersystem nach Anspruch 13, wobei die Kontraktion zwischen dem rechten Atrium und dem linken Ventrikel synchronisiert ist.

16. Schrittmachersystem nach Anspruch 3, wobei das zweite Gehäuse an einem subkutanen Ort implantierbar ist und das Kabel in einem subkutanen Tunnel zwischen dem zweiten Gehäuse und dem ersten Gehäuse angeordnet werden kann.

## Revendications

1. Système de stimulation implantable pour le traitement de l'insuffisance cardiaque, comprenant : au moins un boîtier implantable (10 ; 20 ; 22 ; 30) abritant des circuits configurés pour (a) commander des impulsions de stimulation électrique pour initier au moins une contraction cardiaque, les impulsions étant délivrées en utilisant une ou plusieurs dérivations de stimulation, (b) analyser les signaux cardiaques détectés, les signaux cardiaques étant détectés en utilisant une ou plusieurs dérivations de captage, et (c) activer la transmission d'une énergie vibrationnelle qui est générée par un ou plusieurs transducteurs implantables (14 ; 36) pour stimuler le coeur, les circuits étant adaptés pour commander les impulsions de stimulation électrique et la transmission d'une énergie vibrationnelle dans des conditions choisies pour synchroniser la contraction des chambres cardiaques.

2. Système de stimulation selon la revendication 1, dans lequel les circuits comprennent un premier circuit configuré pour commander l'une ou plusieurs des stimulations électriques, un deuxième circuit configuré pour analyser les signaux cardiaques détectés, et un troisième circuit configuré pour activer la transmission de l'énergie vibrationnelle.

3. Système de stimulation selon la revendication 1, dans lequel au moins le boîtier implantable comprend (a) un premier boîtier (10) comprenant les premier et deuxième circuits, et (b) un deuxième boîtier comprenant le troisième circuit et un ou plusieurs transducteurs vibrationnels, les premier et deuxième boîtiers étant raccordés par un câble.

4. Système de stimulation selon la revendication 3, dans lequel le troisième circuit comprend un amplificateur de puissance, un circuit d'adaptation d'impédances, et un générateur de signal configuré pour activer l'un ou plusieurs des transducteurs vibrationnels.

5. Système de stimulation selon la revendication 3, dans lequel le deuxième circuit comprend en outre des circuits configurés pour générer les impulsions de stimulation électrique et des circuits configurés pour détecter des signaux cardiaques.

6. Système de stimulation selon la revendication 5, dans lequel les circuits de stimulation électrique à impulsions et les circuits de détection se trouvent dans le premier boîtier.

7. Système de stimulation selon la revendication 3, dans lequel au moins l'un des transducteurs vibrationnels est adapté pour être dirigé vers les tissus ventriculaires.

8. Système de stimulation selon la revendication 3, dans lequel au moins l'un des transducteurs vibrationnels est adapté pour être dirigé vers les tissus auriculaires.

9. Système de stimulation selon la revendication 3, dans lequel au moins l'un des transducteurs vibrationnels est adapté pour être dirigé vers les tissus auriculaires ou ventriculaires.

10. Système de stimulation selon la revendication 3, dans lequel au moins l'un des boîtiers implantables est adapté pour comprendre un défibrillateur cardiaque.

11. Système de stimulation selon la revendication 10, dans lequel le défibrillateur cardiaque utilise l'énergie électrique.

12. Système de stimulation selon la revendication 11, dans lequel le défibrillateur cardiaque utilise l'énergie vibrationnelle.

13. Système de stimulation selon la revendication 9, comprenant en outre une ou plusieurs dérivations de stimulation et de captage implantables dans le coeur et raccordées au premier boîtier et raccordées aux premier et deuxième circuits.

14. Système de stimulation selon la revendication 13, dans lequel la contraction est synchronisée entre les ventricules gauche et droit.

15. Système de stimulation selon la revendication 13, dans lequel la contraction est synchronisée entre l'oreillette droite et le ventricule gauche.

16. Système de stimulation selon la revendication 3, dans lequel le deuxième boîtier est implantable dans un site sous-cutané et le câble peut être placé dans un tunnel sous-cutané entre le deuxième boîtier et le premier boîtier.
